(19) Europäisches Patentamt
European Patent Office
Office européen des brevets

(11) EP 1 027 878 A1

(12) DEMANDE DE BREVET EUROPEEN

(43) Date de publication:
**16.08.2000 Bulletin 2000/33**

(51) Int Cl.[7]: **A61K 7/00**, A61K 7/48

(21) Numéro de dépôt: **99403289.4**

(22) Date de dépôt: **27.12.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **05.02.1999 FR 9901387**

(71) Demandeur: **L'OREAL**
**75008 Paris (FR)**

(72) Inventeurs:
- **Ravaux, Danielle**
**78730 Saint-Arnoult en Yvelines (FR)**
- **Laugier, Jean-Pierre**
**92160 Antony (FR)**

(74) Mandataire: **Renard, Emmanuelle**
**L'OREAL-DPI**
**6 rue Bertrand Sincholle**
**92585 Clichy Cedex (FR)**

# (54) Composition cosmétique et/ou dermatologique constituée par une émulsion du type huile-dans-eau formée de vésicules lipidiques dispersées dans une phase aqueuse contenant au moins un actif acide hydrophile

(57) L'invention se rapporte à une composition à usage topique comprenant une émulsion du type huile-dans-eau formée de vésicules lipidiques dispersées dans une phase aqueuse contenant au moins un actif acide hydrophile. Elle est caractérisée en ce que lesdites vésicules sont constituées de globules huileux pourvus chacun d'un enrobage cristal liquide lamellaire obtenu à partir d'au moins un agent tensioactif et en ce que ladite phase aqueuse ne renferme pas de gélifiant.

La composition obtenue a une viscosité supérieure à 500 centipoises à un pH compris entre 2 et 4.

La composition selon l'invention peut éventuellement se présenter sous la forme d'une émulsion, telle que définie ci-dessus, et d'un actif hydrophile pulvérulent destinés à être mélangés extemporanément.

L'invention concerne également un procédé de préparation de cette composition, ainsi que ses utilisations cosmétiques et dermatologiques.

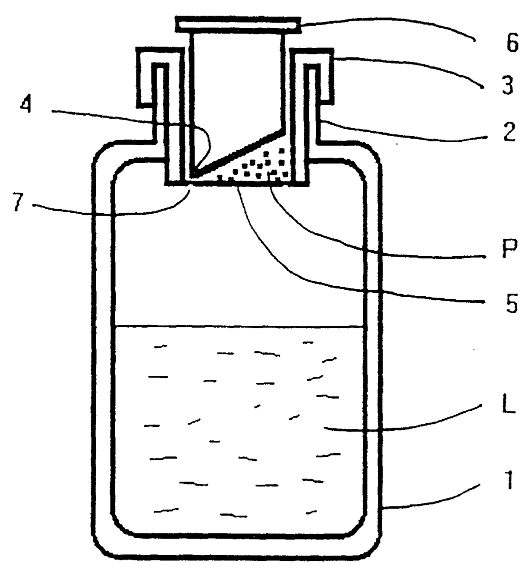


Figure 1

EP 1 027 878 A1

## Description

La présente invention se rapporte à une composition cosmétique et/ou dermatologique à usage topique comprenant une émulsion du type huile-dans-eau formée de vésicules lipidiques dispersées dans une phase aqueuse contenant au moins un actif acide hydrophile.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement comme la lumière, l'oxygène ou l'eau. Il s'ensuit donc une dégradation rapide de l'acide ascorbique formulé en présence de ces paramètres.

Plusieurs solutions ont donc été envisagées dans l'art antérieur pour diminuer et/ou retarder la dégradation de l'acide ascorbique.

Ainsi, EP 0 679 387 divulgue une composition comportant un support hydrophile et de l'acide ascorbique sous forme pulvérulente, destinés à être mélangés extemporanément. Le support est décrit comme comprenant un milieu aqueux et au moins un gélifiant hydrophile et comme étant capable de supporter l'introduction d'un composé acide. Pour ce faire, le gélifiant peut être choisi parmi les polysaccharides, les polymères synthétiques et les celluloses. Un tampon est ajouté à la solution pour que le pH final soit supérieur à 3,8.

Par ailleurs, EP 0 642 781 divulgue une émulsion du type huile-dans-eau de pH inférieur à 3,5, comprenant, à titre d'agent stabilisant, un copolymère anionique réticulé constitué de motifs dérivant de la réaction entre (i) l'acrylamide, (ii) l'acide 2-acrylamido méthyl propane sulfonique (AMPS), et (iii) au moins un composé à polyinsaturation oléfinique. Il est indiqué que le copolymère exerce également, à de plus fortes concentrations, une fonction d'épaississement de l'émulsion, de sorte que l'émulsion est présentée comme pouvant avoir des fluidités diverses allant de celle d'un lait à celle d'une crème.

Enfin, on connaît de EP 0 755 673 une composition topique renfermant au moins un actif sensible à l'eau, tel que l'acide ascorbique, stabilisé par la combinaison d'au moins un polyol et d'au moins un agent structurant choisi parmi les polymères (méth)acryliques et les huiles. Le polyol, qui est par exemple choisi parmi le propylène glycol, les polyéthylène glycols ou la glycérine, est présent en une quantité suffisante pour obtenir une valeur d'activité en eau de la composition inférieure ou égale à 0,85.

Ces compositions de l'art antérieur visant à stabiliser l'acide ascorbique ont pour inconvénient de n'être pas suffisamment visqueuses, voire stables, à pH acide, en particulier à un pH compris entre environ 2 et 4. Or, on pense que le maintien de la composition dans cette gamme de pH garantit qu'une grande partie de l'acide ascorbique restera sous forme protonée, non chargée, dans laquelle la pénétration de la molécule dans la peau est supposée être facilitée. De plus, la quantité élevée de polyol dans la composition décrite dans EP 755 673 lui confère un toucher relativement gras, qui peut ne pas convenir à certaines utilisatrices.

La faible viscosité et/ou stabilité des compositions connues à base d'acide ascorbique et qui comprennent un gélifiant cosmétique tient à ce que, tandis que leur pH diminue, jusqu'à un pH compris entre environ 2 et 4, les gélifiants ioniques qu'elles contiennent subissent des modifications de leur réseau tridimensionnel. On observe en outre une variation des forces de Van der Waals impliquant des gélifiants non ioniques. Il résulte de ces modifications une réduction du pouvoir gélifiant de ces composés, de sorte que l'émulsion renfermant ces gélifiants passe d'une texture crémeuse à la texture d'un fluide tel qu'un lait, voire que les phases aqueuse et huileuse de l'émulsion se séparent, dans des cas extrêmes. La séparation de l'émulsion peut être plus ou moins progressive et/ou plus ou moins complète, selon les cas.

D'autres compositions de l'art antérieur contenant un actif acide hydrophile et un gélifiant cosmétique, et qui posent donc les mêmes problèmes de faible viscosité et/ou stabilité à pH acide, sont celles décrites dans FR-A-2 680 685 et EP 705 593. Il s'agit d'émulsions huile-dans-eau dont la phase huileuse est formée de sphéroïdes lipidiques et dont la phase aqueuse, gélifiée par un composé du type carbomer ou gomme de xanthane, renferme par exemple un filtre UV à fonction acide sulfonique. Ces compositions ont de plus pour inconvénient que les sphéroïdes lipidiques qu'elles contiennent sont des particules solides perceptibles au toucher, d'une taille comprise entre 100 et 5000 µm, qui ont tendance, de par leur taille, à s'écraser à la surface de la peau, ce qui conduit à une pénétration médiocre des actifs qu'elles contiennent.

Un but de la présente invention est donc de proposer une composition contenant un actif acide et susceptible d'avoir une viscosité supérieure à 500 centipoises à pH acide, de préférence à un pH compris entre 2 et 4.

Un autre but de la présente invention est de proposer une composition contenant un actif acide et présentant de bonnes propriétés cosmétiques, en particulier un toucher non gras, c'est-à-dire une composition susceptible d'avoir une valeur d'activité en eau supérieure à 0,90, de préférence supérieure à 0,95.

La Demanderesse a découvert, de manière fortuite, que l'utilisation de vésicules sous forme de phases lamellaires dans des compositions dont la phase aqueuse renferme un actif acide, tel que l'acide ascorbique, et est exempte de gélifiant, permettaient d'atteindre le but précité.

La présente invention a donc pour objet une composition à usage topique, comprenant une émulsion du type huile-dans-eau formée de vésicules lipidiques dispersées dans une phase aqueuse contenant au moins un actif acide hydrophile, les vésicules étant constituées de globules huileux pourvus chacun d'un enrobage cristal liquide lamellaire obtenu à partir d'au moins un tensioactif et ladite phase aqueuse ne renfermant pas de gélifiant.

La composition obtenue a une viscosité supérieure à 500 centipoises. Dans le cadre de cette description, les viscosités indiquées sont celles mesurées à T=10 secondes sur un appareil RHEOMAT RM 180 de METTLER à 25°C, sous pression atmosphérique, à 200 tours/minute, en utilisant un corps de mesure de type ancre appelé "mobile", à savoir le mobile n° 3 fourni avec l'appareil dans le cas des crèmes (contenant l'émulsion et l'actif acide) et le mobile n° 1 dans le cas des laits (contenant l'émulsion sans actif acide). La mesure lue sur l'appareil est convertie en centipoises par report sur une courbe fournie par le constructeur.

L'actif acide peut être choisi parmi les acides organiques hydrophiles, tels que les ∝-hydroxyacides, les β-hydroxyacides et les ∝-cétoacides, éventuellement sous forme lactonisée, et les acides inorganiques tels que l'acide phosphorique. L'actif acide peut également être choisi parmi les acides kojique, caféique, phytique, quinique et l'acide benzène 1,4 - [di (3 -méthylidène camphosulfonique)].

Comme actif acide, on préfère utiliser les acides glycolique, lactique, mandélique, malique, tartrique, citrique, hydroxybutyrique, gluconique, ascorbique, salicylique, gentisique, homogentisique et pyruvique. De préférence, on utilise l'acide ascorbique.

Ledit actif acide est présent, dans la composition selon l'invention, en quantité suffisante pour obtenir l'effet recherché, et qui représente par exemple de 0,1 à 10 % du poids total de ladite composition.

Les vésicules utilisées dans la composition selon l'invention comprennent des globules huileux pourvus chacun d'un enrobage cristal liquide lamellaire.

Selon un mode de réalisation préféré, les globules huileux renferment au moins un actif lipophile.

Ainsi, les globules huileux peuvent renfermer un actif choisi, par exemple, parmi les agents antioxydants, anti-radicaux libres, hydratants, mélanorégulateurs, accélérateurs de bronzage, dépigmentants, de coloration de la peau, liporégulateurs, amincissants, anti-acnéiques, antiséborrhéiques, anti-vieillissement, anti-rides, anti-UV, kératolytiques, anti-inflammatoires, rafraichissants, cicatrisants, protecteurs vasculaires, antibactériens, antifongiques, antiperspirants, déodorants, conditionneurs de la peau, immunomodulateurs, nourrissants, antipelliculaires, anti-chute des cheveux, colorants capillaires, décolorants capillaires, réducteurs pour permanente, conditionneurs des cheveux et les huiles essentielles et les parfums.

On peut citer comme exemples d'actifs lipophiles pour le traitement de la peau et/ou des cheveux, utilisables dans le cadre de la présente invention, les composés suivants : D α tocophérol, DL α tocophérol, acétate de D α tocophérol, acétate DL α tocophérol, palmitate d'ascorbyle, glycérides de vitamine F, vitamines D, rétinol, esters de rétinol, β carotène, D panthénol, farnésol, acétate de farnésyle, huiles riches en acides gras essentiels, acide n octanoyl-5 salicylique, acide salicylique, alkylesters d'α-hydroxyacides, acide asiatique, acide madécassique, asiaticoside, extrait total de Centella Asiatica, acide β glycyrrhétinique, α bisabolol, céramides, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées, huile essentielle de bergamotte, octylmethoxycinnamate, butylméthoxydibenzoyl-méthane, octyl triazone, di tertiobutyl-3,5 hydroxy-4 benzylidène-3 camphre, 2-benzotriazol-2-yl-4-méthyl-6- [3- [1,3,3,3- tétraméthyl- 1- [triméthylsilyl)oxy] disiloxanyl]-2-méthyl-propyl] phénol, huile perfluorée, huile de maïs hyperoxygénée.

Les globules huileux peuvent en outre contenir divers additifs complémentaires tels que huiles, cires ou gommes ayant par exemple des propriétés émollientes ou lubrifiantes.

Les globules huileux enrobés ont avantageusement un diamètre moyen inférieur à 1000 nanomètres, de préférence à 500 nanomètres, mieux, à 200 nanomètres.

L'enrobage cristal liquide lamellaire des globules huileux peut être obtenu, selon une première forme d'exécution, à partir d'au moins un agent tensioactif lipophile, d'au moins un agent tensioactif hydrophile et d'au moins un lipide amphiphile ionique. Les quantités relatives de tensioactifs lipophile, hydrophile et de lipide amphiphile ionique varient de préférence dans les fourchettes respectives suivantes : 35-55% / 25-40% / 15-35% en poids par rapport à leur poids total. Un procédé de préparation de telles vésicules est décrit dans la demande EP 0 705 593.

Dans une seconde forme d'exécution de l'invention, l'enrobage cristal liquide lamellaire peut être obtenu à partir

d'au moins un agent tensioactif lipophile, d'au moins un agent tensioactif hydrophile et d'au moins un acide gras. Dans ce cas, la phase aqueuse contient, à l'état dissous, au moins un agent basique. Les quantités relatives de tensioactif lipophile, hydrophile et d'acide gras varient de préférence dans les fourchettes respectives suivantes : 35-55% / 25-40% / 15-35% en poids par rapport à leur poids total. Un procédé de préparation de telles vésicules est décrit dans la demande EP 0 641 557.

De préférence, l'agent tensioactif lipophile et l'agent tensioactif hydrophile comportent chacun au moins une chaîne grasse saturée ayant plus de 12 atomes de carbone environ, de préférence entre 16 et 22.

En outre, l'agent tensioactif lipophile présente avantageusement un HLB (balance hydrophile-lipophile) compris entre 2 et 5. Des exemples d'agents tensioactifs lipophiles présentant un HLB compris entre 2 et 5 sont le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE (comprenant 2 motifs d'oxyde d'éthylène), le mono et dibéhénate de glycéryle, le tétrastéarate de pentaérythritol.

De son côté, l'agent tensioactif hydrophile présente avantageusement un HLB compris entre 8 et 12. Des exemples d'agents tensioactifs hydrophiles présentant un HLB compris entre 8 et 12 sont le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE, le distéarate de méthylglucose polyoxyéthyléné 20 OE.

Le lipide amphiphile ionique peut être choisi dans le groupe comprenant les lipides anioniques neutralisés, les lipides amphotères et les dérivés alkylsulfoniques. Par exemple, le lipide amphiphile ionique peut être choisi dans le groupe comprenant les sels alcalins du dicétylphosphate, les sels alcalins du dimyristylphosphate, les sels alcalins du cholestérol sulfate, les sels alcalins du cholestérol phosphate, les sels mono et disodiques des acides acylglutamiques, les phospholipides, les dérivés alkylsulfoniques de formule :

$$R\text{-}CH\text{-}CO\text{-}O\text{-}(CH_2CH_2O)_2\text{-}CH_3$$
$$|$$
$$SO_3M$$

dans laquelle R représente les radicaux $C_{16}H_{33}$ et $C_{18}H_{37}$ pris en mélange ou séparément et M est un métal alcalin.

L'acide gras peut être choisi dans le groupe comprenant l'acide palmitique, l'acide stéarique, l'acide arachidique et l'acide béhénique.

L'agent basique est dissous dans la phase aqueuse en une quantité au moins égale à la quantité nécessaire à la neutralisation de l'acide gras. Il peut être choisi dans le groupe comprenant la soude, la triéthanolamine, la lysine et l'arginine.

Les compositions de l'invention peuvent encore contenir dans la phase aqueuse divers additifs complémentaires tels que des agents conservateurs ou des agents séquestrants.

Les compositions se présentent le plus fréquemment sous forme de lait ou de crème, d'autres modes de présentation n'étant pas exclus.

La composition selon l'invention peut se présenter sous la forme :

- d'une émulsion du type huile-dans-eau formée de vésicules lipidiques dispersées dans une phase aqueuse ne renfermant pas de gélifiant, lesdites vésicules étant constituées de globules huileux pourvus chacun d'un enrobage cristal liquide lamellaire obtenu à partir d'au moins un agent tensioactif ; et
- d'au moins un actif acide sous forme pulvérulente ou en solution aqueuse, destinés à être mélangés extemporanément.

Dans ce cas, l'émulsion est par exemple conditionnée dans un récipient fermé par un bouchon renfermant l'actif acide, un séparateur perforable et/ou détachable étant prévu entre l'actif et l'émulsion.

La figure 1 représente, en coupe longitudinale schématique, un exemple de flacon bi-compartimenté, étanche à la lumière, contenant la composition selon l'invention, avant mélange de l'émulsion et de l'actif pulvérulent, tel que de l'acide ascorbique pur à au moins 99%.

Précisément, le flacon est composé d'un corps (1) en un matériau faisant écran à la lumière extérieure, et faisant office de réservoir pour l'émulsion fluide (L). L'émulsion (L) a une viscosité inférieure à 10 centipoises. A sa

partie supérieure, le réservoir (1) est muni d'un col (2) dans lequel est inséré un godet (3), fermé à sa partie inférieure par un opercule (5), godet à l'intérieur duquel est placé l'actif (P). L'opercule (5) peut être moulé d'une seule pièce avec le godet (3) et comporte de préférence des zones de faiblesse. En variante, l'opercule (5) peut être constitué d'une feuille d'aluminium thermosoudée sur la partie inférieure du godet.

Dans la partie supérieure ouverte du godet (3) est inséré, de façon étanche, un trocard (6) muni, à sa partie inférieure, d'une zone tranchante (4) capable de découper l'opercule (5). Avant utilisation, le godet (3) muni de son trocard (6) fait office de bouchon au récipient.

Le fonctionnement de l'ensemble est le suivant.

L'utilisateur enfonce le trocard (6) dans le godet (5), ce qui provoque la déchirure de l'opercule (5) le long de ses lignes de faiblesse et l'écoulement de l'actif pulvérulent (P) dans l'émulsion fluide (L). L'utilisateur agite ensuite l'ensemble pour dissoudre l'actif dans l'émulsion qui atteint alors une viscosité d'au moins 500 centipoises. Il retire ensuite le trocard (6) du godet (3) pour libérer l'orifice de distribution et place éventuellement une tétine en plastique mou, percée à son extrémité, sur le godet (3), en vue de faciliter la libération du produit sur la peau.

D'autres exemples de flacons à compartiments multiples, qui peuvent être utilisés pour conditionner séparément l'émulsion et l'actif pulvérulent selon la présente invention sont notamment ceux décrits dans EP 0 528 707, EP 0 230 195, FR 84 13355 et FR 85 17143, sans que cette liste ne soit limitative.

La composition selon l'invention peut être appliquée sur la peau, les poils ou les cheveux, suivant l'usage auquel elle est destinée. Elle peut ainsi être utilisée dans une préparation cosmétique et/ou dans un procédé de traitement cosmétique de la peau, consistant à appliquer la composition selon l'invention sur la peau, par exemple en vue de la tonifier, de la régénérer, de lisser les ridules de la peau, et/ou pour lutter contre les méfaits des rayonnements UV et/ou pour renforcer les tissus cutanés contre les agressions de l'environnement.

En variante, la composition selon l'invention peut être utilisée dans un procédé de traitement thérapeutique, c'est-à-dire pour la fabrication d'une préparation dermatologique, telle qu'une préparation destinée à blanchir et/ou dépigmenter la peau, les poils et/ou les cheveux.

La présente invention concerne encore un procédé de préparation d'une composition contenant un actif acide hydrophile, ladite composition ayant une viscosité supérieure à 500 centipoises à un pH compris entre 2 et 4, comprenant l'introduction dudit actif dans une émulsion du type huile-dans-eau formée de globules huileux dispersés dans une phase aqueuse ne renfermant pas de gélifiant, lesdits globules huileux étant pourvus chacun d'un enrobage cristal liquide lamellaire obtenu à partir d'au moins un tensioactif.

Il est donné ci-après des exemples de compositions conformes à l'invention. Les quantités sont indiquées en pourcentage pondéral, sauf indication contraire.

**EXEMPLES**

**Exemple 1**

Dans un flacon à double compartiment du type de celui illustré à la figure 1, on introduit l'émulsion suivante :

| | |
|---|---|
| Polyglycéryl 2-stéarate | 0,2 g |
| PEG-8 stéarate | 0,135 g |
| Sel disodique de suif hydrogéné et du L-acide glutamique (Amisoft HS-20 fourni par AJINOMOTO) | 0,09 g |
| Isocétyl stéarate | 0,7 g |
| Squalane | 1,3 g |
| Eau | 7,075 g |
| TOTAL | 9,5 g |

L'émulsion se présente sous la forme d'un lait fluide blanc ayant une viscosité d'environ 7 centipoises à 25°C et un pH de 7,3 ± 0,3.

Le flacon est serti d'une capsule contenant 0,5 g d'acide ascorbique sous forme de poudre blanche.

Lors de l'introduction de l'acide pulvérulent dans l'émulsion ci-dessus, on observe une diminution du pH et une augmentation substantielle de la viscosité, jusqu'à obtenir une composition ayant une viscosité de 850 centipoises à 25°C, revêtant l'aspect d'une crème blanche, et un pH de 3,3 ± 0,3.

Après dix jours à température ambiante, on n'observe pas de diminution de la quantité d'acide ascorbique

présente en solution (mesurée par chromatographie liquide haute pression). Le pH de la crème n'est pas modifié.

**Exemple 2**

Dans un flacon à double compartiment du type de celui illustré à la figure 1, on introduit l'émulsion suivante :

| | |
|---|---|
| Polysorbate 61 | 0,16 g |
| Acide stéarique | 0,12 g |
| Alcool stéarylique | 0,11 g |
| Vaseline | 0,3 g |
| Cholestérol | 0,005 g |
| Tristéarate de sucrose | 0,264 g |
| Isocétyl stéarate | 0,5 g |
| Cyclopentasiloxane | 0,3 g |
| Eau | 7,681 g |
| Triéthanolamine | 0,06 g |
| TOTAL | 9,5 g |

L'émulsion se présente sous la forme d'un lait fluide blanc ayant une viscosité d'environ 6 centipoises à 25°C et un pH de 7,7 ± 0,3.
Le flacon est serti d'une capsule contenant 0,5 g d'acide ascorbique sous forme de poudre blanche.
Après introduction de l'acide pulvérulent dans l'émulsion ci-dessus, on obtient une crème blanche ayant une viscosité de 1300 centipoises à 25°C et un pH de 3,5 ± 0,3.
Après dix jours à température ambiante, la crème présente un très léger relargage qui peut être évité en la conservant plutôt à 4°C. Son pH n'est pas modifié.

**Exemple 3**

Dans un flacon à double compartiment du type de celui illustré à la figure 1, on introduit l'émulsion suivante :

| | |
|---|---|
| Polyglycéryl 2-stéarate | 0,2 g |
| PEG-8 stéarate | 0,135 g |
| Sel disodique de suif hydrogéné et du L-acide glutamique (Amisoft HS-20 fourni par AJINOMOTO) | 0,09 g |
| Isocétyl stéarate | 0,7 g |
| Squalane | 1,3 g |
| Eau | 7,075 g |
| TOTAL | 9,5 g |

L'émulsion se présente sous la forme d'un lait fluide blanc ayant une viscosité d'environ 7 centipoises à 25°C et un pH de 7,3.
Sur le flacon est sertie une capsule renfermant 0,5 g d'acide glycolique pulvérulent.
Après introduction de l'acide pulvérulent dans l'émulsion ci-dessus, on obtient une crème blanche ayant une viscosité de 600 centipoises à 25°C et un pH de 2,5.

**Exemple 4**

Dans un flacon à double compartiment du type de celui illustré à la figure 1, on introduit l'émulsion suivante :

| | |
|---|---|
| Polysorbate 61 | 0,16 g |

(suite)

| | |
|---|---|
| Acide stéarique | 0,12 g |
| Alcool stéarylique | 0,11 g |
| Vaseline | 0,3 g |
| Cholestérol | 0,005 g |
| Tristéarate de sucrose | 0,264 g |
| Isocétyl stéarate | 0,5 g |
| Cyclopentasiloxane | 0,3 g |
| Eau | 7,681 g |
| Triéthanolamine | 0,06 g |
| TOTAL | 9,5 g |

Sur le flacon est sertie une capsule renfermant 0,5 g d'acide phosphorique en solution à 85% dans l'eau, destiné à être mélangé extemporanément à l'émulsion ci-dessus.

**Exemple 5**

Dans le flacon à double compartiment du type de celui illustré à la figure 1, on introduit l'émulsion suivante :

| | |
|---|---|
| Polysorbate 61 | 0,16 g |
| Acide stéarique | 0,12 g |
| Alcool stéarylique | 0,11 g |
| Vaseline | 0,3 g |
| Cholestérol | 0,005 g |
| Tristéarate de sucrose | 0,264 g |
| Isocétyl stéarate | 0,5 g |
| Cyclopentasiloxane | 0,3 g |
| Eau | 7,681 g |
| Triéthanolamine | 0.06 g |
| TOTAL | 9,5 g |

Sur le flacon est sertie une capsule renfermant 0,5 g d'acide lactique en solution à 90% dans l'eau. La solution d'acide lactique et l'émulsion ci-dessus sont destinées à être mélangées extemporanément pour obtenir une crème de jour.

**Exemple 6 : mesure de la valeur d'activité en eau ($a_w$) de la composition**

L'activité en eau $a_w$ d'un milieu contenant de l'eau est le rapport de la pression de vapeur d'eau du produit « $P_{H2O}$ produit » et de la pression de vapeur de l'eau pure « $P_{H2O}$ pur » à la même température. Elle peut être exprimée aussi comme le rapport du nombre de molécules d'eau « $N_{H2O}$ » sur le nombre de molécules totales « $N_{H2O}$ + $N_{corps\ dissous}$ », qui tient compte de celles des corps dissous « $N_{corps\ dissous}$ ».

Elle est donnée par les formules suivantes :

$$a_w = \frac{P_{H2O}\ \text{produit}}{P_{H2O}\ \text{pur}} = \frac{N_{H2O}}{N_{H2O} + N_{corps\ dissous}}$$

On peut utiliser différentes méthodes pour mesurer l'activité en eau. La plus courante est la méthode manométrique par laquelle on mesure directement la pression de vapeur.

<u>Résultats</u> : les valeurs d'activité en eau des crèmes des Exemples 1 et 2 ci-dessus ont été déterminées comme étant respectivement de 0,988 et 0,991. Ces valeurs traduisent les bonnes propriétés cosmétiques de ces crèmes, qui sont dépourvues du toucher caractéristique des crèmes de l'art antérieur stabilisées par des polyols.

**Revendications**

1. Composition à usage topique comprenant une émulsion du type huile-dans-eau formée de vésicules lipidiques dispersées dans une phase aqueuse contenant au moins un actif acide hydrophile, caractérisée en ce que lesdites vésicules sont constituées de globules huileux pourvus chacun d'un enrobage cristal liquide lamellaire obtenu à partir d'au moins un agent tensioactif et en ce que ladite phase aqueuse ne renferme pas de gélifiant.

2. Composition selon la revendication 1, caractérisée en ce que ledit actif acide est choisi parmi les acides organiques hydrophiles tels que les α-hydroxyacides, les β-hydroxyacides et les α-cétoacides, éventuellement sous forme lactonisée, et les acides inorganiques tels que l'acide phosphorique.

3. Composition selon la revendication 2, caractérisée en ce que ledit actif acide est choisi parmi les acides glycolique, lactique, mandélique, malique, tartrique, citrique, hydroxybutyrique, gluconique, ascorbique, salicylique, gentisique, homogentisique et pyruvique.

4. Composition selon la revendication 3, caractérisée en ce que ledit actif acide est l'acide ascorbique.

5. Composition selon la revendication 2, caractérisée en ce que ledit actif acide est choisi parmi les acides kojique, caféique, phytique, quinique et l'acide benzène 1,4 - [di (3 -méthylidène camphosulfonique)].

6. Composition selon l'une quelconque des revendications précédentes, caractérisée en ce que ledit enrobage cristal liquide lamellaire est obtenu à partir d'au moins un agent tensioactif lipophile, d'au moins un agent tensioactif hydrophile et d'au moins un lipide amphiphile ionique.

7. Composition selon l'une quelconque des revendications 1 à 5, caractérisée en ce que ledit enrobage cristal liquide lamellaire est obtenu à partir d'au moins un agent tensioactif lipophile, d'au moins un agent tensioactif hydrophile et d'au moins un acide gras, et en ce que la phase aqueuse contient, à l'état dissous, au moins un agent basique.

8. Composition selon la revendication 6 ou 7, caractérisée en ce que l'agent tensioactif lipophile et l'agent tensioactif hydrophile comportent chacun au moins une chaine grasse saturée ayant plus de 12 atomes de carbone environ, de préférence entre 16 et 22.

9. Composition selon la revendication 6 ou 7, caractérisée en ce que l'agent tensioactif lipophile présente un HLB (balance hydrophile-lipophile) compris entre 2 et 5.

10. Composition selon la revendication 9, caractérisée en ce que l'agent tensioactif lipophile présentant un HLB compris entre 2 et 5 est choisi dans le groupe comprenant le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE (comprenant 2 motifs d'oxyde d'éthylène), le mono et dibéhénate de glycéryle, le tétrastéarate de pentaérythritol.

11. Composition selon la revendication 6 ou 7, caractérisée en ce que l'agent tensioactif hydrophile présente un HLB compris entre 8 et 12.

12. Composition selon la revendication 6 ou 7, caractérisée en ce que l'agent tensioactif hydrophile présentant un HLB compris entre 8 et 12 est choisi dans le groupe comprenant le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE, le distéarate de méthylglucose polyoxyéthyléné 20 OE.

13. Composition selon la revendication 6, caractérisée en ce que le lipide amphiphile ionique est choisi dans le groupe comprenant les lipides anioniques neutralisés, les lipides amphotères et les dérivés alkylsulfoniques.

14. Composition selon la revendication 6, caractérisée en ce que le lipide amphiphile ionique est choisi parmi le groupe comprenant les sels alcalins du dicétylphosphate, les sels alcalins du dimyristylphosphate, les sels alcalins du cholestérol sulfate, les sels alcalins du cholestérol phosphate, les sels mono et disodiques des acides acylglutamiques, les phospholipides, les dérivés alkylsulfoniques de formule :

$$\begin{array}{l} R\text{-}CH\text{-}CO\text{-}O\text{-}(CH_2CH_2O)_2\text{-}CH_3 \\ \quad\;\; | \\ \quad\; SO_3M \end{array}$$

dans laquelle R représente les radicaux $C_{16}H_{33}$ et $C_{18}H_{37}$ pris en mélange ou séparément et M est un métal alcalin.

**15.** Composition selon la revendication 7, caractérisée en ce que l'acide gras est choisi dans le groupe comprenant l'acide palmitique, l'acide stéarique, l'acide arachidique et l'acide béhénique.

**16.** Composition selon la revendication 7, caractérisée en ce que l'agent basique est dissous dans la phase aqueuse en une quantité au moins égale à la quantité nécessaire à la neutralisation de l'acide gras.

**17.** Composition selon la revendication 7, caractérisé en ce que l'agent basique est choisi dans le groupe comprenant la soude, la triéthanolamine, la lysine et l'arginine.

**18.** Composition selon l'une quelconque des revendications précédentes, caractérisée en ce que lesdits globules huileux renferment au moins un actif lipophile.

**19.** Composition selon la revendication 18, caractérisée en ce que ledit actif lipophile est choisi parmi les agents antioxydants, anti-radicaux libres, hydratants, mélanorégulateurs, accélérateurs de bronzage, dépigmentants, de coloration de la peau, liporégulateurs, amincissants, anti-acnéiques, antiséborrhéiques, anti-vieillissement, anti-rides, anti-UV, kératolytiques, anti-inflammatoires, rafraichissants, cicatrisants, protecteurs vasculaires, antibactériens, antifongiques, antiperspirants, déodorants, conditionneurs de la peau, immunomodulateurs, nourrissants, antipelliculaires, anti-chute des cheveux, colorants capillaires, décolorants capillaires, réducteurs pour permanente, conditionneurs des cheveux et les huiles essentielles et les parfums.

**20.** Composition selon l'une quelconque des revendications 18 à 19, caractérisée en ce que ledit actif lipophile est choisi parmi : D $\alpha$ tocophérol, DL $\alpha$ tocophérol, acétate de D $\alpha$ tocophérol, acétate DL $\alpha$ tocophérol, palmitate d'ascorbyle, glycérides de vitamine F, vitamines D, rétinol, esters de rétinol, $\beta$ carotène, D panthénol, farnésol, acétate de farnésyle, huiles riches en acides gras essentiels, acide n octanoyl-5 salicylique, acide salicylique, alkylesters d'$\alpha$-hydroxyacides, acide asiatique, acide madécassique, asiaticoside, extrait total de Centella Asiatica, acide $\beta$ glycyrrhétinique, $\alpha$ bisabolol, céramides, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées, huile essentielle de bergamotte, octylmethoxycinnamate, butylméthoxydibenzoyl-méthane, octyl triazone, di tertiobutyl-3,5 hydroxy-4 benzylidène-3 camphre, 2-benzotriazol-2-yl-4-méthyl-6-[3-[1,3,3,3-tétraméthyl-1-[triméthylsilyl)oxy]disiloxanyl]-2-méthylpropyl] phénol, huile perfluorée, huile de maïs hyperoxygénée.

**21.** Composition selon l'une quelconque des revendications précédentes, caractérisée en ce que ledit actif acide représente de 0,1 à 10 % du poids total de ladite composition.

**22.** Composition selon l'une quelconque des revendications précédentes, caractérisée en ce qu'elle se présente sous la forme :

- d'une émulsion du type huile-dans-eau formée de vésicules lipidiques dispersées dans une phase aqueuse ne renfermant pas de gélifiant, lesdites vésicules étant constituées de globules huileux pourvus chacun d'un enrobage cristal liquide lamellaire obtenu à partir d'au moins un agent tensioactif, et
- d'au moins un actif acide sous forme pulvérulente ou en solution aqueuse,

destinés à être mélangés extemporanément.

**23.** Composition selon la revendication 22, caractérisée en ce que l'émulsion (L) est conditionnée dans un récipient (1) fermé par un bouchon (3) renfermant l'actif acide (P), un séparateur perforable et/ou détachable (5) étant prévu entre l'actif (P) et l'émulsion (L).

**24.** Composition selon la revendication 22 ou 23, caractérisée en ce qu'avant mélange avec l'actif acide, l'émulsion a une viscosité inférieure à 10 centipoises.

**25.** Procédé de préparation d'une composition contenant un actif acide hydrophile, ladite composition ayant une viscosité supérieure à 500 centipoises à un pH compris entre 2 et 4, comprenant l'introduction dudit actif dans une émulsion du type huile-dans-eau formée de globules huileux dispersés dans une phase aqueuse ne renfermant pas de gélifiant, lesdits globules huileux étant pourvus chacun d'un enrobage cristal liquide lamellaire obtenu à partir d'au moins un tensioactif.

**26.** Utilisation de la composition selon l'une quelconque des revendications 1 à 24 dans une préparation cosmétique.

**27.** Utilisation de la composition selon l'une quelconque des revendications 1 à 24 dans un procédé de traitement cosmétique de la peau, en vue de la tonifier, de la régénérer, de lisser les ridules de la peau, et/ou pour lutter contre les méfaits des rayonnements UV et/ou pour renforcer les tissus cutanés contre les agressions de l'environnement.

**28.** Procédé de traitement cosmétique, caractérisé en ce qu'il consiste à appliquer sur la peau une composition selon l'une quelconque des revendications 1 à 24.

**29.** Utilisation de la composition selon l'une quelconque des revendications 1 à 24 pour la fabrication d'une préparation dermatologique telle qu'une préparation blanchissante et/ou dépigmentante.

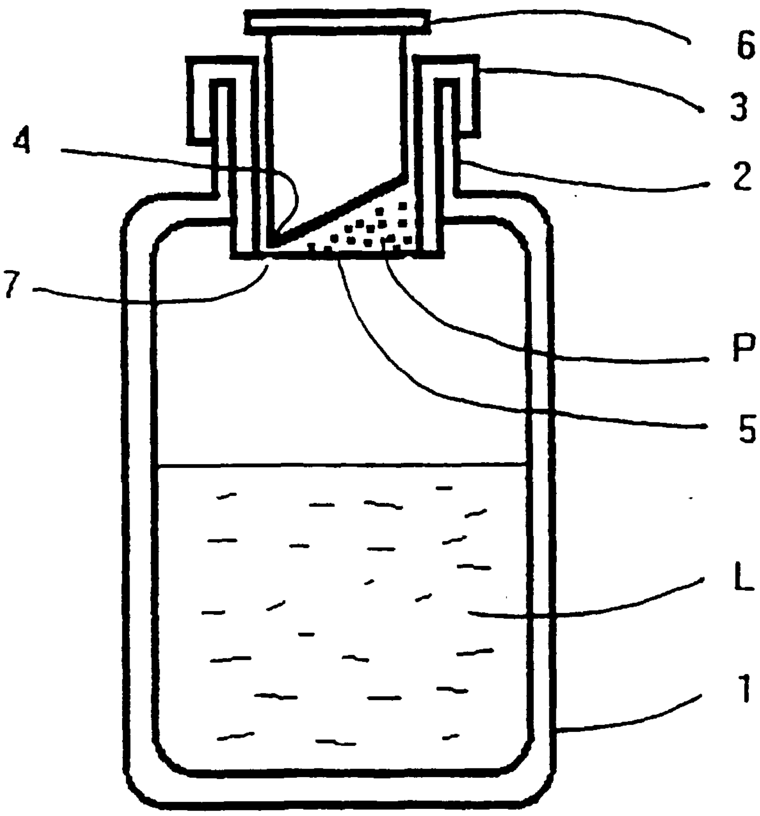


Figure 1

Office européen des brevets

# RAPPORT DE RECHERCHE EUROPEENNE

Numéro de la demande
EP 99 40 3289

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | PATENT ABSTRACTS OF JAPAN vol. 5, no. 200, 18 décembre 1981 (1981-12-18) & JP 56 120612 A (KANEBO KESHOHIN), 22 septembre 1981 (1981-09-22) * abrégé * | 1,4 | A61K7/00 A61K7/48 |
| X | US 5 585 109 A (J. A. HAYWARD ET AL) 17 décembre 1996 (1996-12-17) * revendication 1 * | 1,3 | |
| D,A | EP 0 705 593 A (L'OREAL) 10 avril 1996 (1996-04-10) * revendications 1,3-9,21 * | 1,8-14, 20 | |
| D,A | EP 0 679 387 A (L'OREAL) 2 novembre 1995 (1995-11-02) * revendication 1 * | 22 | |
| D,A | EP 0 755 673 A (L'OREAL) 29 janvier 1997 (1997-01-29) * revendications 1,12 * | 1,4 | DOMAINES TECHNIQUES RECHERCHES (Int.Cl.7) |
| A | DATABASE WPI Week 199125 Derwent Publications Ltd., London, GB; AN 1991-183200 XP002122916 "PH-sensitive liposome(s) - with wall membrane contg. N-acylhomoserine and/or N-acylhomoserine lactone, and which opt. contain drugs" & JP 03 112924 A (AGENCY OF IND. SCI. & TECHNOLOGY), 14 mai 1991 (1991-05-14) * abrégé * | 1,4 | A61K |

-/--

Le présent rapport a été établi pour toutes les revendications

| Lieu de la recherche | Date d'achèvement de la recherche | Examinateur |
|---|---|---|
| LA HAYE | 26 mai 2000 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 99 40 3289

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 729 746 A (UNILEVER) 4 septembre 1996 (1996-09-04) * revendication 1 * | 22 | |
| A | US 4 818 521 A (H. TAMABUCHI) 4 avril 1989 (1989-04-04) * revendication 1 * | 1,4 | |
| A | US 5 531 993 A (J. GRIAT) 2 juillet 1996 (1996-07-02) * revendication 1; exemples 1,2 * | 1,3 | |
| A | DE 197 22 405 A (IFAC) 3 décembre 1998 (1998-12-03) * revendications 1,3,5 * | 1,3 | |
| A | DE 42 38 779 A (MAX-DELBRÜCK-CENTRUM) 19 mai 1994 (1994-05-19) * revendications 1-6 * | 1,4 | |
| A | EP 0 771 557 A (L'OREAL) 7 mai 1997 (1997-05-07) * revendications 1,9 * | 1,4 | DOMAINES TECHNIQUES RECHERCHES (Int.Cl.7) |
| P,A | WO 99 36053 A (COLOR ACCESS) 22 juillet 1999 (1999-07-22) * revendications 1,3,6,7,9 * | 1-4 | |

Le présent rapport a été établi pour toutes les revendications

| Lieu de la recherche | Date d'achèvement de la recherche | Examinateur |
|---|---|---|
| LA HAYE | 26 mai 2000 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 99 40 3289

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

26-05-2000

| Document brevet cité au rapport de recherche | Date de publication | Membre(s) de la famille de brevet(s) | Date de publication |
|---|---|---|---|
| JP 56120612 A | 22-09-1981 | AUCUN | |
| US 5585109 A | 17-12-1996 | AT 192327 T | 15-05-2000 |
| | | AU 673023 B | 24-10-1996 |
| | | AU 5314194 A | 29-09-1994 |
| | | EP 0616799 A | 28-09-1994 |
| | | FI 940993 A | 25-09-1994 |
| | | JP 6279230 A | 04-10-1994 |
| | | NO 940249 A | 26-09-1994 |
| | | NZ 250660 A | 21-12-1995 |
| | | SG 65599 A | 22-06-1999 |
| | | ZA 9309802 A | 18-08-1994 |
| EP 705593 A | 10-04-1996 | FR 2725369 A | 12-04-1996 |
| | | AT 144409 T | 15-11-1996 |
| | | BR 9504781 A | 15-10-1996 |
| | | CA 2160071 A | 08-04-1996 |
| | | DE 69500077 D | 28-11-1996 |
| | | DE 69500077 T | 20-02-1997 |
| | | ES 2096505 T | 01-03-1997 |
| | | JP 2650881 B | 10-09-1997 |
| | | JP 8127526 A | 21-05-1996 |
| | | PL 310848 A | 15-04-1996 |
| | | US 5925364 A | 20-07-1999 |
| EP 679387 A | 02-11-1995 | FR 2718018 A | 06-10-1995 |
| | | AT 151279 T | 15-04-1997 |
| | | BR 9501319 A | 05-03-1996 |
| | | CA 2146298 A | 06-10-1995 |
| | | CN 1113143 A | 13-12-1995 |
| | | DE 69500220 D | 15-05-1997 |
| | | DE 69500220 T | 07-08-1997 |
| | | DE 679387 T | 13-03-1997 |
| | | ES 2095818 T | 01-03-1997 |
| | | HU 71723 A,B | 29-01-1996 |
| | | JP 2651366 B | 10-09-1997 |
| | | JP 7277919 A | 24-10-1995 |
| | | KR 178377 B | 01-04-1999 |
| | | PL 307990 A | 16-10-1995 |
| | | RU 2114602 C | 10-07-1998 |
| | | US 6010706 A | 04-01-2000 |
| EP 755673 A | 29-01-1997 | FR 2737116 A | 31-01-1997 |
| | | BR 9604009 A | 22-04-1998 |
| | | DE 69601755 D | 22-04-1999 |
| | | DE 69601755 T | 15-07-1999 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 99 40 3289

La présente annexe indique les membres de la famille de brevets relatifs aux documents brevets cités dans le rapport de recherche européenne visé ci-dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

26-05-2000

| Document brevet cité au rapport de recherche | Date de publication | Membre(s) de la famille de brevet(s) | Date de publication |
|---|---|---|---|
| EP 755673 A | | ES 2133907 T | 16-09-1999 |
| | | JP 9040543 A | 10-02-1997 |
| | | US 5703041 A | 30-12-1997 |
| JP 3112924 A | 14-05-1991 | JP 1869016 C | 06-09-1994 |
| | | JP 5079644 B | 04-11-1993 |
| EP 729746 A | 04-09-1996 | AUCUN | |
| US 4818521 A | 04-04-1989 | AUCUN | |
| US 5531993 A | 02-07-1996 | FR 2709982 A | 24-03-1995 |
| | | AT 175864 T | 15-02-1999 |
| | | CA 2132144 A | 16-03-1995 |
| | | DE 69416060 D | 04-03-1999 |
| | | DE 69416060 T | 27-05-1999 |
| | | EP 0642781 A | 15-03-1995 |
| | | ES 2129602 T | 16-06-1999 |
| | | JP 2693116 B | 24-12-1997 |
| | | JP 7163859 A | 27-06-1995 |
| | | US 6001379 A | 14-12-1999 |
| | | US 5863545 A | 26-01-1999 |
| DE 19722405 A | 03-12-1998 | WO 9853797 A | 03-12-1998 |
| DE 4238779 A | 19-05-1994 | AUCUN | |
| EP 771557 A | 07-05-1997 | FR 2738743 A | 21-03-1997 |
| | | AT 183383 T | 15-09-1999 |
| | | DE 69603814 D | 23-09-1999 |
| | | DE 69603814 T | 09-12-1999 |
| | | ES 2138306 T | 01-01-2000 |
| | | JP 9110628 A | 28-04-1997 |
| WO 9936053 A | 22-07-1999 | AU 1201099 A | 02-08-1999 |

EPO FORM P0460

Pour tout renseignement concernant cette annexe : voir Journal Officiel de l'Office européen des brevets, No.12/82